# EUROPEAN PATENT SPECIFICATION

(11) **EP 4 308 248 B1**
(45) Date of publication and mention of the grant of the patent: **06.05.2026**
(21) Application number: 22712564.8
(22) Date of filing: 10.03.2022
(51) Int. Cl.: A61Q 19/08, A61K 8/37, A61K 8/67

(54) **COSMETIC SKIN CARE COMPOSITION**
KOSMETISCHE HAUTPFLEGEZUSAMMENSETZUNG
COMPOSITION COSMÉTIQUE DE SOINS DE LA PEAU

(30) Priority: 16.03.2021 EP 21162783
(43) Date of publication of application: 24.01.2024
(73) Proprietor: Unilever IP Holdings B.V., 6708 WH Wageningen (NL); Unilever Global IP Limited, Wirral, Merseyside CH62 4ZD (GB)
(72) Inventor: AU, Van, Trumbull, Connecticut 06611 (US); DAVIS, Andrew, John, Trumbull, Connecticut 06611 (US); GUELAKIS, Marian, Pereira, Trumbull, Connecticut 06611 (US); HARICHIAN, Bijan, deceased (ZZ); LATHROP, William, F., Trumbull, Connecticut 06611 (US); LEE, Jianming, Trumbull, Connecticut 06611 (US); LU, Nandou, Trumbull, Connecticut 06611 (US); ROSA, Jose, Guillermo, Trumbull, Connecticut 06611 (US)
(74) Representative: Unilever Patent Group
(86) International application number: PCT/EP2022/056114
(87) International publication number: WO 2022/194653

(56) References cited:
- WO-A1-2015/072909
- DE-A1- 19 843 566
- DE-A1- 2 748 399
- US-A- 4 219 548

## Description

### FIELD OF THE INVENTION

This disclosure relates to topical cosmetic compositions for application on human skin, as well as to their use in improving the condition and appearance of skin.

### BACKGROUND OF THE INVENTION

Skin is subject to deterioration through dermatological disorders, environmental abuse (wind, air conditioning, central heating, pollution) or through the normal ageing process (chronoageing) which may be accelerated by exposure of skin to sun (photoageing). The demand for cosmetically improving the appearance and condition of human skin is universal and timeless. In particular, the enormous demand is for reversing, reducing or preventing the visible signs of wrinkled, aged and/or photodamaged skin. Consumers everywhere are continually seeking "anti-ageing" cosmetic products that reverse, treat or delay the visible signs of chronoaging and photoaging skin such as wrinkles, lines, sagging, hyperpigmentation and age spots.

Collagen, the predominant matrix skin protein is known to impart tensile strength to skin. Levels of collagen in skin are significantly reduced with aged and/or photodamaged skin, i.e., collagen fibrils break down and do not receive mechano-transduction signals (see definition below). Many studies have shown that the levels of collagen type I in skin is decreased with age and/or with increased photodamage (for example Lavker, R.J. Inv. Derm., (1979, 73,79-66; and Griffiths et al. N. Eng. J. Med.(1993) 329, 530-535). The reduction of the levels of collagen in skin is associated with a decrease in the tensile strength of the skin, i.e., structural damage in aged skin, causing and appearing as wrinkles and laxity. Conversely, strengthening of the dermal matrix by boosting the level of collagen in skin provides anti-ageing/dermal repair benefits.

The extracelluar matrix (ECM) once thought to function only as scaffold to structurally support cells, regulates many aspects of cell behavior, including morphology, proliferation, differentiation, and survival. Through the process of mechano-transduction, cells can convert mechanical stimuli into biochemical or transcriptional changes [Chiquet, M., et al., From mechanotransduction to extracellular matrix gene expression in fibroblasts. Biochim Biophys Acta, 2009. 1793(5): p. 911-20.

Daley, W.P., S.B. Peters, and M. Larsen, Extracellular matrix dynamics in development and regenerative medicine. J Cell Sci, 2008. 121(Pt 3): p. 255-64.] This signal transduction involves proteins of the ECM, the cytoplasmic membrane, the cytoskeleton and the nuclear membrane, eventually affecting the nuclear chromatin at a genetic and epigenetic level.

Mechano-transduction signaling is a key driver of cell behavior. The physical and mechanical properties of the cellular microenvironment regulate cell shape and can strongly influence cellular phenotype. The recent identification of YAP (Yes-associated protein) and TAZ (transcriptional co-activator with PDZ-binding motif), both transcriptional effectors of the Hippo pathway, as mechano-sensors and mechano-transducers, has begun to shed some light on the mechanism for cells sensing and transducing mechanical cues to regulate gene expression.

See Mohri, Z., A. Del Rio Hernandez, and R. Krams, The emerging role of YAP/TAZ in mechanotransduction. J Thorac Dis, 2017. 9(5): p. E507-E509.

Dupont, S., Role of YAP/TAZ in cell-matrix adhesion-mediated signalling and mechanotransduction. Exp Cell Res, 2016. 343(1): p. 42-53.

Halder, G., S. Dupont, and S. Piccolo, Transduction of mechanical and cytoskeletal cues by YAP and TAZ. Nat Rev Mol Cell Biol, 2012. 13(9): p. 591-600.

Zhang, H., H.A. Pasolli, and E. Fuchs, Yes-associated protein (YAP) transcriptional coactivator functions in balancing growth and differentiation in skin. Proc Natl Acad Sci U S A, 2011. 108(6): p. 2270-5.

Dupont, S., et al., Role of YAPlTAZ in mechanotransduction. Nature, 2011. 474(7350): p. 179-83.

YAP and TAZ have been shown to function as key mechano-sensors and mechano-transducers, translating a broad range of mechanical cues from sheer stress and cell shape to ECM rigidity into cell-specific transcriptional programs.

Proper dermal ECM mechanical properties sustain youthful fibroblast behavior. In young skin, fibroblast in the dermis are well spread, engaging via integrins, with the abundant and organized bundles of collagen fibrils. With (photo)ageing, collagen fibrils become fragmented and disorganized. Fibroblasts lose collagen (and other ECM) adhesion sites, resulting in a collapsed morphology, decreased TGFb signaling and production of collagen, and increased production of matrix-degrading enzymes. In this aged microenvironment, there is compromised force homeostasis between the cells and the fibrils of ECM, resulting in deficient mechano-transduction signaling [Fisher, G.J., et al.,

Reduction of fibroblast size/mechanical force down-regulates TGF-beta type II receptor: implications for human skin aging. Aging Cell, 2016. 15(1): p. 67-76.

Qin, Z., et al., Age-associated reduction of cellular spreading/mechanical force upregulates matrix metalloproteinase-1 expression and collagen fibril fragmentation via c-Jun/AP-1 in human dermal fibroblasts. Aging Cell, 2014. 13(6): p. 1028-37.

Qin, Z., et al., Oxidant exposure induces cysteine-rich protein 61 (CCN1) via c- Jun/AP-1 to reduce collagen expression in human dermal fibroblasts. PLoS One, 2014. 9(12): p. e115402.

Fisher, G.J., D.L. Sachs, and J.J. Voorhees, Ageing: collagenase-mediated collagen fragmentation as a rejuvenation target. Br J Dermatol, 2014. 171(3): p. 446-9.

Fisher, G.J., J. Varani, and J.J. Voorhees, Looking older: Fibroblast collapse and therapeutic implications. Archives of Dermatology, 2008. 144(5): p. 666-672.]. Moreover, enhancing the mechanical properties of the dermis with a filler of cross-linked hyaluronic has been shown to activate skin rejuvenation activity; injection of non-crosslinked material did not stimulate the same bioactivity See Quan, T., et al., Enhancing structural support of the dermal microenvironment activates fibroblasts, endothelial cells, and keratinocytes in aged human skin in vivo. J Invest Dermatol, 2013. 133(3): p. 658-67.

Therefore, Applicant hypothesized that activation of YAP via topical application to skin may override the deficient mechanical signaling from aged dermis to drive skin rejuvenation behavior.

While YAP/TAZ are being explored for tissue regeneration of various tissues such as heart and intestine [Hong, A.W., Z. Meng, and K.L. Guan, The Hippo pathway in intestinal regeneration and disease. Nat Rev Gastroenterol Hepatol, 2016. 13(6): p. 324-37.

Lin, Z. and W.T. Pu, Harnessing Hippo in the heart: Hippo/Yap signaling and applications to heart regeneration and rejuvenation. Stem Cell Res, 2014. 13(3 Pt B): p. 571-81.

Moya, I.M. and G. Halder, The Hippo pathway in cellular reprogramming and regeneration of different organs. Curr Opin Cell Biol, 2016. 43: p. 62-68. 18. Wang, Y., A. Yu, and F.X. Yu, The Hippo pathway in tissue homeostasis and regeneration. Protein Cell, 2017. 8(5): p. 349-359.

Xiao, Y., et al., Hippo/Yap Signaling in Cardiac Development and Regeneration. Curr Treat Options Cardiovasc Med, 2016. 18(6): p. 38.]

Applicant in its research has unexpectedly discovered the power of YAP/TAZ in driving skin rejuvenation activity and actives that can significantly boost it.

Applicant in its research has shown that activation of YAP on soft hydrogel model system elicits procollagen production as well as activation of other biomarkers of ECM-building activity; together these data suggest that activation of YAP may improve the appearance of skin via ECM building bioactivity.

The intracellular localization of YAP/TAZ is a key determinant in the regulation of its activity and their roles in signal transduction. YAP/TAZ shuttle between the cellular nucleus and the cytoplasm. When YAP is located in the nucleus it is considered active, helping to drive the transcription of genes critical for tissue growth. When YAP is sequestered in the cytoplasm, it is rendered inactive, as it cannot drive gene transcription activity in that compartment of the cell. Thus, the activation of signal transduction mechanisms leading to collagen production may be achieved via mechano-transduction pathways. YAP is needed in skin cell nucleus in order to drive production of collagen. YAP/TAZ pathway is a mechanotransdution signaling pathway that exists in human cells that both senses the mechanical signals of the extracellular space and responds to these signals by driving gene expression activity to direct consequent cellular behavior. YAP is used as a marker of tissue regeneration. Based on published literature, Applicant developed a nuclear translocation assay - done with routine tissue culture capabilities and using antibodies for YAP. The assay shows nuclear translocation between the cytoplasm and the nucleus and measures the percent accumulation in the nucleus. High throughput assays are available from proprietors, such as Cytoo, Grenoble, France.

Applicant has added gene expression and pro-collagen studies as confirmation of the downstream consequences or applications of YAP nuclear translocation assay results. Applicant has unexpectedly identified cosmetic rejuvenation actives for topical application to the skin which, without wishing to be bound by theory, boost the YAP activated pathways in skin cells.

Applicant has now found that effective treatment and prevention of normal, but cosmetically undesirable, skin conditions, due to chronoaging or photoaging, such as wrinkles, lines, sagging, hyperpigmentation and age spots, may be obtained through the application of cosmetic compositions to the skin which comprise specific monounsaturated fatty acid esters of glycerol. The monoglycerides according to the present invention have a role in the mechano-transduction pathway.

Lysophosphatidic acid (LPA) represents a fatty acid monoglyceride phosphate monoester where the fatty acid chain of the monoglyceride group is comprised of saturated fatty acids (mainly palmitoyl and stearoyl) as well as unsaturated fatty acids (mainly linoleoyl, arachidonyl and oleoyl). Although LPA has been scarcely used for a limited number of skin care products, these type of phosphate monoesters have significant drawbacks that limit their utility across a broader range of personal care products. For instance, LPA is not sufficiently stable to survive long term shelf-life criteria in formulated products, especially at elevated temperatures. In addition, the polar phosphate headgroup of LPA renders it a surfactant molecule and prevents efficient delivery through the skin to reach functional levels at the biological target. Furthermore, the accessibility of large-scale LPA supply is challenging and substantially expensive to allow broad usage across various product categories. Hence, there is a need for more commercially feasible and chemically stable compounds with acceptable skin delivery profiles that can provide LPA-like benefits to the skin.

Structure-function relationship studies on LPA have demonstrated that a fatty acid alkyl group containing more than 12 carbon atoms and a phosphate monoester group are both required for LPA-induced biological activity. However, Applicant has surprisingly discovered that certain compounds lacking the phosphate ester headgroup (e.g. specific monoglycerides) exhibit comparable LPA-like functionality in the mechano-transduction pathway. Applicant has also discovered that the observed mechano-transduction signaling is sensitive to the structure of the fatty acid group of the monoglyceride. The compounds according to the present invention also provide significant advantages over LPA-like compounds for personal care products including large scale supply, inexpensive sourcing and better chemical stability and skin delivery profiles.

Certain glycerin fatty acid derivatives for skin barrier recovery, mainly glyceryl monooleate and glyceryl monostearate (referred to herein as "GMS") are described in KR2015/112578. See also DE19843566 A1 to Beiersdorf.

Glyceryl monostearate (GMS) and oleoyl-rac-glycerol (ORG) are usually used as emulsifiers at levels of up to 5% or as carriers for pharmaceutical compositions. In KR1015566552B1, oleic acid or stearic acid monoacylglycerol is used as a function oil for cooking oil, for metabolic disease, or as a lipid for cosmetics.

The art discussed above does not disclose the specific fatty acid ester of glycerol selected from cis-mono-unsaturated fatty acid ester of glycerol on the 1- or 2- position of the glycerol backbone for treating ageing skin. Nor does the above art disclose any links between such glycerol esters and any biological targets leading to any beneficial effects/consequences or modes of action on skin cellular level.

### SUMMARY OF THE INVENTION

Applicant has identified a specific fatty acid ester of glycerol selected from cis-mono-unsaturated fatty acid ester of glycerol on the 1- or 2- position of the glycerol backbone for treating ageing skin.

According to a first aspect of the present invention there is provided a topical composition comprising:
(a) a cosmetic skin benefit agent selected from 1-oleoyl-rac-glycerol, (2S)-1-oleoylglycerol, oleoyl-rac-glycerol (ORG), (2R)-1-oleoylglycerol, 2-oleoylglycerol, and/or mixtures thereof;
(b) a retinoid, wherein the retinoid is retinyl propionate; and
(c) a dermatologically acceptable vehicle.

The present invention encompasses the use of the inventive compositions comprising cis-mono-unsaturated fatty acid ester of glycerol on the 1- or 2- position of the glycerol backbone, preferably oleoyl-rac-glycerol ("ORG") and oils rich in ORG, in skin care compositions for providing at least one skin care benefit selected from treating/preventing wrinkling, sagging, aged and/or photodamaged skin; boosting collagen deposition in skin, boosting mechano-transduction in skin cells, enhancing tissue repair; and improving skin texture, smoothness and/or firmness; and promoting skin barrier recovery.

The present invention encompasses a cosmetic method of providing at least one cosmetic skin care benefit selected from:
treating/preventing wrinkling, sagging, dry, aged and/or photodamaged skin;
boosting/maintaining collagen levels in skin, boosting/maintaining decorin levels in skin, enhancing tissue repair; improving skin texture, smoothness and/or firmness; promoting skin barrier recovery;
the method comprising applying to a skin a topical composition as described above.

According to a second aspect of the present invention there is provided a cosmetic method of providing at least one skin care benefit selected from:
treating/preventing wrinkling, sagging, dry, aged and/or photodamaged skin;
boosting collagen deposition in skin, boosting mechano-transduction in skin cells, enhancing tissue repair; improving skin texture, smoothness and/or firmness; and promoting skin barrier recovery;
the method comprising applying to the skin a topical composition as described above.

According to a third aspect of the present invention there is provided a cosmetic use of oleoyl-rac-glycerol ("ORG") in combination with retinyl propionate as an activator of YAP activated receptors in a topical composition for providing at least one skin care benefit selected from treating/preventing wrinkling, sagging, aged and/or photodamaged skin; boosting/maintaining collagen deposition in skin, boosting/maintaining decor in production in skin, enhancing tissue repair; improving skin texture, and smoothness and/or firmness, and/or promoting skin barrier recovery.

**The** inventive compositions, methods and uses thus provide anti-ageing benefits which result in the promotion of smooth and supple skin with improved elasticity and a reduced or delayed appearance of wrinkles and aged skin, with improved more even skin colour. A general improvement in the appearance, texture and condition, in particular with respect to the radiance, clarity, and general youthful appearance of skin is achieved. The term "cosmetic" as used herein includes within its scope normal but visibly undesirable appearance of skin as perceived by a consumer.

The term "dermal matrix" as used herein includes within its scope the dermal extracellular matrix ("ECM") in the dermal layer of the skin where major structural damage occurs with ageing.

The term "extracellular matrix" or "extracellular dermal matrix" or "ECM" as used herein includes within its scope its function as scaffold to structurally support cells. Without wishing to be bound by theory, Applicant believes that the ECM additionally regulates many aspects of cell behavior, including morphology, proliferation, differentiation, and survival. Through the process of mechano-transduction, cells can convert mechanical stimuli into biochemical or transcriptional changes. This signal transduction involves proteins of the ECM, the cytoplasmic membrane, the cytoskeleton and the nuclear membrane, eventually affecting the nuclear chromatin at a genetic and epigenetic level.

The term "leave-on" as used herein includes within its scope leaving on skin for a prolonged period of time before washing off.

The term "topical" as used herein includes within its scope application to the exterior of the skin.

The term "treating" as used herein includes within its scope cosmetically or visibly reducing, delaying and/or preventing the above mentioned normal, but cosmetically undesirable, skin conditions caused by the normal ageing process. The visible signs of aging, such as wrinkles, lines and/or sagging are delayed or reduced. Generally, the quality of skin is enhanced and its appearance and texture is improved by preventing or reducing wrinkling and increasing flexibility, firmness, smoothness, suppleness and elasticity of the skin. The compositions, methods and uses according to the invention may be useful for treating skin that is already in a wrinkled, aged, and/or photodamaged condition or for treating youthful skin to prevent or reduce those aforementioned undesirable changes due to the normal ageing/photoageing process.

Mechanotransduction is a mechanism by which cells convert mechanical stimulus into electrochemical activity. The term "mechnotransduction" as used herein includes within its scope a signaling mechanism for how cells perceive and relay mechanical information from the ECM to the nucleus. Mechano-transduction signaling has become increasingly evident as a key driver of cell behavior. Recently, two transcriptional effectors of the Hippo signaling pathway, YAP and TAZ, have been shown to function as key mechano-sensors and mechano-transducers, translating a broad range of mechanical cues from sheer stress and ECM rigidity into cell-specific transcriptional programs.

The term GMS as used here is short for Glyceryl Mono Stearate.

The term oleoyl-rac-gycerol or ORG, as used herein, refers to a combination of 1-oleoyl-rac-glycerol (or oleic acid monoglyceride, or 1-cis conjugated oleic acid monoglyceride) and has the chemical structure below: 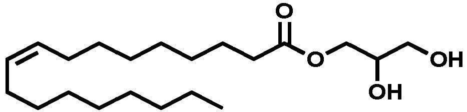 and 2-oleoylglycerol (or 2-cis conjugated oleic acid monoglyceride or 2-oleoyl-rac-glycerol) which has the chemical structure below:

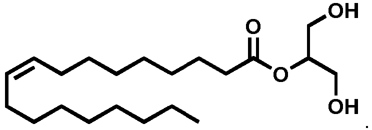

ORG may also refer to glyceryl monooleate (or glyceryl oleate or oleoyl monoglyceride) which has a combination of 1-oleoyl-rac-glycerol and 2-oleoylglycerol.

In an embodiment, ORG has from about 5 to 99%, or 15% to about 99%, or about 25% to about 99%, or about 35% to about 99%, or about 45% to about 99%, or about 50% to about 99%, or about 55% to about 99%, or about 65% to about 99%, or about 75% to about 99%, or about 85% to about 99%, or about 90% to about 99%, or about 95% to 99%, or about 5% to about 95%, or about 15% to about 95%, or about 25% to about 95%, or about 35% to about 95%, or about 45% to about 95%, or about 50% to about 95%, or about 55% to about 95%, or about 65% to about 95%, or about 75% to about 95%, or about 85% to about 95%, or about 90% to about 95%, 5% to about 90%, or about 15% to about 90%, or about 25% to about 90%, or about 35% to about 90%, or about 45% to about 90%, or about 50% to about 90%, or about 55% to about 90%, or about 65% to about 90%, or about 75% to about 90%, or about 85% to about 90%, by weight of 1-oleoyl-rac-glycerol, including all ranges subsumed therein.

The term "YAP" as used herein includes within its scope the Yes-associated protein. The term "TAZ" as used herein includes within its scope the transcriptional co-activator with PDX binding motif. Both are transcriptional effectors of the Hippo pathway, as mechano-sensors and mechano-transducers. The "Hippo pathway" as used herein includes within its scope a highly conserved signaling network that controls cell proliferation, differentiation, and cell death.

### Detailed Description of the Invention

### Oleic Acid and its Triglyceride Ester

Glycerol, also known as 1,2,3-propanetriol or triglyceride, has a 3-carbon backbone with 3 OH groups. The OH-groups are easily esterified naturally or synthetically to yield triglyceride ester derivatives, or triglycerides, all positional isomers substituted with fatty acids on the glycerol backbone. See Fessenden, et al., Organic Chemistry, Chapter 19, p. 870 (1979). Said triglycerides (glycerol backbone) must contain at least one fatty acid moiety. For example, of the three esterifiable positions on the glycerol backbone, according to the present invention, the 1 and/or 2 positions may be esterified with oleic acid and by another lipid, preferably with a lipid at position 1, more preferably with the lipid having a cis monounsaturation, and most preferably with and oleic acid on the 1-position of the glycerol backbone.

Oleic acid (hereinafter referred to as OA) is a cis-monounsaturated long chain (C18) fatty acid derived from animal and/or plant fat, having the formula
CH₃(CH₂)₇CH=CH(CH₂)₇COOH. See Fessenden, et al., Organic Chemistry, Chapter 19, p. 870 (1979).

The invention includes derivatives of the free acid which thus comprise oleic acid moieties. Preferable derivatives include those derived from substitution of the carboxyl group of the acid, such as esters (e.g. triglyceride esters, monoglyceride esters, diglyceride esters, phosphoesters), amides (e.g. ceramide derivatives), salts (e.g. alkali metal and alkali earth metal salts, ammonium salts); and/or those derived from substitution of the C18/ C22 carbon chain, such as alpha hydroxy and/or beta hydroxy derivatives, as well as derivatives containing hydroxy groups further down the fatty acid chain away from the carboxyl groups such as 12-hydroxystearic acid and ricinoleic acid derivatives. Preferably, cis-mono-unsaturated fatty acid ester of glycerol on the 1- or 2-position of the glycerol backbone (e.g., 1-oleoyl-rac-glycerol and/or 2-oleoylglycerol), preferably ORG and oils rich in ORG.

Oils that are rich in ORG are also suitable for use in the present invention. Such oils are commercially available and include MONOMULS^{®} brand from BASF, containing about 90% glyceryl monooleate (having a combination of 1-oleoyl-rac-glycerol and 2-oleoylglycerol), with the balance comprising di- and tri- esters and free glycerol.

### COSMETIC ACTIVATORS OF YAP NUCLEAR ACCUMULATION

The activity of YAP/TAZ is determined by its localization within the cell; nuclear YAP/TAZ is active while cytosolic YAP/TAZ protein is inactive. The goal is to induce YAP to shuttle from the cytoplasm to the skin cell nucleus thereby eliciting activation of mechanotransduction signaling pathways. YAP in the nucleus promotes skin cell proliferation/tissue growth. YAP/TAZ is a marker of rejuvenation.

Applicant has unexpectedly discovered that ORG is a particularly effective activator of YAP/TAZ nuclear accumulation and/or is a signaling molecule for mechano-transduction pathway. Applicant has discovered and shown that ORG is a uniquely effective activator of YAP/TAZ bioactivity in cells which reside in the deeper layers of skin ; YAP/TAZ activity has been previously described to regulate behavior of cells in top layer of skin ( keratinocyte biology) There is no disclosure or suggestion in the art of ORG being an activator of YAP/TAZ or driving YAP to the skin cell nuceli, thereby enhancing mechanotransduction signals and driving production of collagen from fibroblasts in dermal layer, or having use in cosmetic compositions for providing cosmetic anti-ageing treatments.

An established and widely accepted method by which YAP activation can be demonstrated and thus by which activators of YAP can be identified is the nuclear translocation assay. Cosmetic materials that are activators of YAP are thus easily identifiable by those skilled in the art as those compounds which cause the nuclear accumulation of YAP, outlined in Example 1 below.

Thus, if a cosmetic compound passes this in vitro YAP assay, that is, it drives YAP to the skin cell nucleus in the assay outlined in Example 1 below, it is included as a lipid YAP activator even if it is not specifically mentioned herein. In a preferred embodiment of the invention, the lipid YAP activator is a compound which promotes accumulation of YAP in the nucleus above the levels achieved by the vehicle control, as these are the more effective anti-ageing agents.

Applicant has developed a hydrogel-based model of tunable substrate rigidity to study YAP/TAZ mechano-transduction signaling in the context of dermal rejuvenation, and Applicant has provided proof of science for the importance of YAP in driving skin rejuvenation activity.

A cosmetic skin benefit agent, as used herein, is a YAP activator, selected from 1-oleoyl-rac-glycerol, (2S)-1-oleoylglycerol, oleoyl-rac-glycerol (ORG), (2R)-1-oleoylglycerol, 2-oleoylglycerol, and/or mixtures thereof.

The fatty acid moieties may be straight or branched chain, unsaturated and may be substituted e.g. 12-hydroxy fatty acid.

Preferred YAP activators according to the invention include 1-oleoyl-rac-glycerol, (2S)-1-oleoylglycerol, oleoyl-rac-glycerol (ORG), (2R)-1-oleoylglycerol, 2-oleoylglycerol, and/or mixtures thereof. In an embodiment, the YAP activator is 1-oleoyl-rac-glycerol. In an embodiment, the YAP activator is 2-oleoylglycerol.

It should also be understood that the YAP activator which is present in compositions according to the invention is ideally present in the "active" form; that is, it is not esterified as a di- or tri-glyceride. As such, while natural sources of the material such as oils are referred to above, the YAP activator which is used in compositions according to the invention is preferably not the raw, esterified form of the activator, but rather a raw material source which is either rich in the unesterified YAP activator, or one in which the esterified form comprises a di- or tri-glyceride derived solely from oleic acid or a di- or triglyceride derived from oleic acid and mixtures of a C6-C22 fatty acid which preferentially releases the "active" form.

Without wishing to be bound by theory, Applicant believes that the linear carbon chain structure of long chain (e.g., C16-C22) saturated fatty acid glycerides (e.g., such as the C18 carbon chain found in 1-stearoyl-rac-glycerol) is detrimental to YAP activating potency compared to a corresponding cis-mono-unsaturated fatty acid carbon chain (e.g., such as the C18:1 cis carbon chain present in ORG). The nature of the linear and saturated long carbon chains of such fatty acid derivatives allows these molecules to stack together via strong Van der Waals intermolecular forces, , resulting in greater amounts on energy required to break them apart compared to fatty acid derivatives with cis-mono-unsaturated carbon chains. Consequentially, Applicant also believes that the solubility and dermal delivery of ORG is better than that of saturated derivatives 1-stearoyl-rac-glycerol or 1-lauroyl-rac-glycerol.

YAP activators, including ORG, are employed in the inventive composition in an amount of between 0.0001% and 50%, or between 0.001% and 10%, or between 0.01% to 10% by weight of the composition. More preferably, the amount is from 0.01% to 5% or from 0.1% to 5%, or from 0.1% to 0.4%, and most preferably from 0.1% to 3% in order to maximise benefits at a minimum cost; even more preferably with a lower limit about 1.15% to about 1.5% and upper limit of about 2.0% to about 3.0%. Preferably, it is present at a level of less than 4% by weight; with preferred examples including 1.5%, 2.0% and 3.0%.

### Dermatologically Acceptable Vehicle

The composition used according to the invention also comprises a dermatologically/cosmetically acceptable vehicle to act as a dilutant, dispersant or carrier for the actives. The vehicle may comprise materials commonly employed in skin care products such as water, liquid or solid emollients, silicone oils, emulsifiers, solvents, humectants, thickeners, powders, propellants and the like.

The vehicle will usually form from 5% to 99.9%, preferably from 25% to 80% by weight of the composition, and can, in the absence of other cosmetic adjuncts, form the balance of the composition.

### Optional Skin Benefit Materials and Cosmetic Adjuncts

Besides the selected cosmetic skin benefit actives, other specific skin-benefit actives such as additional anti-aging actives, e.g. retinoids including but not limited to retinyl propionate, phosphatidic acid derivatives including but not limited to lysophosphatidic acid (LPA) and cyclic phosphatidic acid (cLPA, or otherwise known as CyPA^{®}, sunscreens, skin-lightening agents, skin tanning agents may also be included. Retinyl propionate is particularly advantageous. Moisturization actives may include petroselinic acid, ricinoleic, linoleic acid, linolenic acid, oleic acid and arachidonic acid -- useful for forming desired fatty acid esters of glycerol. The vehicle may also further include adjuncts such as antioxidants, perfumes, opacifiers, preservatives, colorants and buffers.

Retinoids, such as retinyl propionate, are employed in the inventive composition in an amount from about 0.001% to 10%, or about 0.001% to about 5%, or about 0.001% to about 2%, or about 0.001% to about 1%, or about 0.001% to about 0.5%, or about 0.01% to about 5%, or about 0.01% to about 3%, or about 0.01% to about 1.5%, or about 0.01% to about 1%, or about 0.01% to about 0.5% by weight of the topical composition, including all ranges subsumed therein.

Phosphatidic acid derivatives, as described herein, are employed in the inventive composition in an amount from about 0.001% to 10%, or about 0.001% to about 5%, or about 0.001% to about 3%, or about 0.001% to about 1%, or about 0.001% to about 0.5%, or about 0.01% to about 5%, or about 0.01% to about 3%, or about 0.01% to about 1.5%, or about 0.01% to about 1%, or about 0.01% to about 0.5% by weight of the topical composition, including all ranges subsumed therein.

In an aspect of the invention, a weight ratio of YAP activators to retinoids is from 20:1 to 1:1, or from 10:1 to 1:1, or from 9:1 to 1:1, or from 8:1 to 1:1, or from 7:1 to 1:1, or from 6:1 to 1:1, or from 5:1 to 1:1, or from 4:1 to 1:1, or from 3:1 to 1:1.

In an aspect of the invention, a molar ratio of YAP activators to retinoids is from 20:1 to 1:1, or from 10:1 to 1:1, or from 9:1 to 1:1, or from 8:1 to 1:1, or from 7:1 to 1:1, or from 6:1 to 1:1, or from 5:1 to 1:1, or from 4:1 to 1:1, or from 3:1 to 1:1.

### Product Preparation, Form, Use and Packaging

To prepare the topical composition used in the method of the present invention, the usual manner for preparing skin care products may be employed. The active components are generally incorporated in a dermatologically/cosmetically acceptable carrier in conventional manner. The active components can suitably first be dissolved or dispersed in a portion of the water or another solvent or liquid to be incorporated in the composition. The preferred compositions are oil-in-water or water-in-oil or water-in-oil-in-water emulsions.

The composition may be in the form of conventional skin-care products such as a cream, gel or lotion, capsules or the like. Most preferably the product is a "leave-on" product; that is, a product to be applied to the skin without a deliberate rinsing step soon after its application to the skin.

The composition may be packaged in any suitable manner such as in a jar, a bottle, tube, roll-ball, or the like, in the conventional manner. It is also envisaged that the inventive compositions could be packaged as a kit of two separate compositions, one containing the lipid component OA of the present invention and the second containing an optional skin benefit agent component, to be applied to the skin simultaneously or consecutively or at separate times.

The method of the present invention may be carried out one or more times daily to the skin which requires treatment. The improvement in skin appearance will usually become visible after 3 to 6 months, depending on skin condition, the concentration of the active components used in the inventive method, the amount of composition used and the frequency with which it is applied. In general, a small quantity of the composition, for example from 0.1 to 5 ml is applied to the skin from a suitable container or applicator and spread over and/or rubbed into the skin using the hands or fingers or a suitable device. A rinsing step may optionally follow depending on whether the composition is formulated as a "leave-on" or a "rinse-off" product.

In order that the present invention may be more readily understood, the following examples are given, by way of illustration only.

### EXAMPLES

### Example 1. Nuclear Translocation Assay to Identify Activators of YAP/TAZ Bioactivity

Cellular localization of YAP can be regulated by the rigidity of the substrate or ECM that cells are attached to, or the degree to which cells can spread on a substrate. Thus in vitro models can be useful to engineer low states of YAP activity. An established and widely accepted method by which YAP/TAZ activation can be demonstrated has been published.

Briefly, primary human dermal fibroblasts were seeded on microprinted substrate islands (< 1000 µm²) of fibronectin and incubated overnight at 37°C, 5% CO₂ in a cell culture incubator. Next, fibroblasts were treated with materials described in Table 1. After the treatment period of 2 hours, cells were fixed and stained: nuclei were stained with Hoechst dye and YAP/TAZ was stained with a mouse antibody to YAP/TAZ (Santa Cruz Biotechnology), followed by the secondary fluorescently tagged antibody (donkey antimouse AlexaFluor 488). Microscopic images were acquired and YAP/TAZ fluorescent signal measured in the cytoplasm and nucleus of each cell. The fluorescence intensity measured in the nucleus is divided by the one measured in the cytoplasm to obtain the YAP/TAZ ratio readout as described below Table 1.

Cosmetic materials which activate YAP/TAZ are identified.
A. The following experimental data supports advantages of the monounsaturated glycerol ester 1-oleoyl-rac-glycerol over other saturated glycerol esters by recording accumulation of YAP in the cell nucleus. Accumulation of YAP in the nucleus is used as a read-out of Mechano-transduction activation.

**Table 1.**

| Effect of Alkyl Chain of Fatty Acid Glycerol Esters on Mechano-transduction | | |
|---|---|---|
| Test Sample | Structure | Mechanotransduction Activation (% Increase) * |
| DMSO | 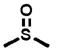 | 0-3 |
| Lysophosphatidic-Acid "LPA" (Positive Control) | 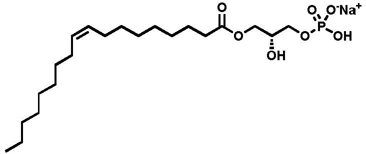 | 100 +/- 1 ** |
| 1-oleoyl-rac-glycerol | 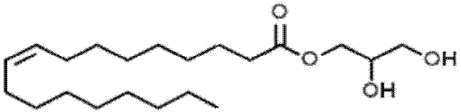 | 17 +/- 10 ** |
| 1-Stearoyl-rac-glycerol | 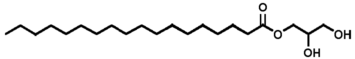 | 6 +/- 7 |
| 1-Pentadecanoyl-rac-glycerol | 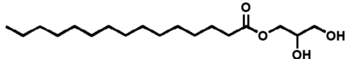 | 25 +/- 26 |

| | | |
|---|---|---|
| * Mechano-transduction activation (YAP/TAZ localization from cytoplasm to nucleus) is expressed as: % = [(ratio _{test compound}) - (avg. ratio _{control}) / (avg. ratio _{LPA}) - (avg. ratio _{control})] * 100. LPA and test compounds were tested at 20uM ** Statistically significant over Control. | | |

As can be seen from the data in Table 1 above, the positive control lysophosphatidic acid (LPA) induces mechano-transduction signaling (i.e., translocation of cytoplasmic YAP/TAZ into the nucleus) over the vehicle DMSO. Surprisingly, the monounsaturated fatty acid glycerol ester, 1-oleoyl-rac-glycerol, also significantly induces mechanotransduction signaling. In contrast, the saturated fatty acid glycerol esters, 1-stearoyl-rac-glycerol or 1-pentadecanoyl-rac-glycerol, do not induce mechano-transduction. Hence, mono-unsaturation of long chain fatty acid glycerol esters is necessary to activate the mechano-transduction YAP/TAZ signaling pathway. Moreover and unexpectedly, attaching a (-)-charged phosphate anionic headgroup to a long chain monounsaturated fatty acid glycerol ester is not essential for triggering such mechano-transduction signaling.

### B. Delivery and Deposition

The dermal delivery of 1-oleoyl-rac-glycerol and 1-stearoyl-rac-glycerol from topically applied formulations was predicted using a mathematical model. In this dermal delivery model, skin is treated as having three layers: stratum corneum, viable epidermis and dermis, each layer with a typical thickness as reported in the literature (15, 85, 1000 mm, respectively). The formulation containing the test compound is treated as another layer at the skin surface. This dermal delivery model simulates the dynamic process of the test compound penetration into the skin based on a partition-diffusion theory: in each skin and product layer, the mass transport of the test compound is characterized using the Fick's 2^{nd} law of diffusion, while the transfer of the test compound at the interface between two adjacent layers is governed by partitioning. The potential binding of the test compound in each skin layer is also simulated. The key input parameters of the dermal delivery model include the molecular weight, water-octanol partition coefficient, chemical formulation (number of each type of atoms and double-bonds). The mathematical equations, method of the estimation of parameters (including diffusivities, partition coefficients and binding coefficients) from the model input parameters were described in a paper by Dancik et al. (Yuri Dancik, Mattew A. Miller, Joanna Jaworska, Gerald B. Kasting, Design and performance of a spreadsheet-based model for estimating bioavailability of chemicals from dermal exposure. Advanced Drug Delivery Reviews, 65:221-236, 2013.) The present model, however, refined the estimation of the partition coefficient between the product and the stratum corneum to better predict the delivery of test compounds from typical skin formulations.

The predicted dermal delivery of 1-oleoyl-rac-glycerol and 1-stearoyl-rac-glycerol into the viable epidermis and dermis at 24-hour post topical application are presented in Table 2.

The formulations were assumed to be applied at a level of 2 mg/cm2 which is typical in clinical studies. The amount of delivery is presented in terms of the percent of the total amount of the test compound (% Dose) contained in the applied formulation. Note that the predicted results remain unchanged for formulations containing up to 5 wt-% of the test compound, and at different application levels (e.g., 1 or 5 mg/cm2.)

**Table 2. Dermal Delivery Prediction:**

| 1-Oleoyl-rac-glycerol versus 1-Stearoyl-rac-glycerol | | |
|---|---|---|
| Test Compound | % Dose Delivered at 24hr * | |
| | Viable Epidermis | Dermis |
| 1-Oleoyl-rac-glycerol | 0.0184 | 0.0166 |
| 1-Stearoyl-rac-glycerol | 0.0060 | 0.0054 |

| | | |
|---|---|---|
| * Formulations were assumed to be applied at a level of 2 mg test compound /cm2 skin | | |

As illustrated from the data above in Table 2, 1-oleoyl-rac-glycerol was predicted to deliver much better than 1-stearoyl-rac-glycerol (at least 3X better) through the skin to the epidermal and dermal layers from a typical skin formulation, and the latter predicted to deliver much better than lysophosphatidic acids such as LPA. Further, functional levels of 1-oleoyl-rac-glycerol (but not 1-stearoyl-rac-glycerol) are predicted to deliver to these skin layers from a skin formulation containing 3% 1-oleoyl-rac-glycerol, which corresponds to levels of fatty acid glycerol esters found in some skin products. Therefore, 1-oleoyl-rac-glycerol is not only capable of inducing mechanotransduction, but also has the right physicochemical properties (over saturated glycerol esters and lysophosphatidic acids) to allow effective delivery to the epidermal and dermal layers where it is needed to activate mechano-transduction pathways.

### C. This Example demonstrates that Water solubility is another advantage of 1-oleoyl-rac-glycerol over 1-stearoyl-rac-glycerol for cosmetic applications

**Table 3. Water Solubility Prediction of 1-Oleoyl-rac-glycerol versus 1-Stearoyl-rac-glycerol.**

| Test Compound | Aqueous Solubility (mol/L)* |
|---|---|
| 1-Oleoyl-rac-glycerol | 5.13 X 10-8 |
| 1-Stearoyl-rac-glycerol | 2.88 X 10-8 |

| | |
|---|---|
| * Calculated using Aqueous Solubility (logS) Predictor available from ChemAxon Co. | |

As shown from the data above in Table 3, 1-oleoyl-rac-glycerol was predicted to be almost 2X more soluble in aqueous media over 1-stearoyl-rac-glycerol which makes the former more suitable and more versatile for a wider range of cosmetic formulations for topical application. Further, the melting point of 1-oleoyl-rac-glycerol (i.e., 35 °C) is below body temperature (i.e., 37 °C), ensuring that once it is applied to the skin (in a formulation), it will remain in the liquid state and able to penetrate and deliver through the skin as intended. In contrast, the melting point of 1-stearoyl-rac-glycerol is above body temperature (i.e., 40.5 °C), suffering from the possibility of premature precipitation on top of the skin and significantly hindering dermal delivery. Hence, the greater aqueous solubility, in combination with its lower melting point (below body temperature) for 1-oleoyl-rac-glycerol makes it a better choice over 1-stearoyl-rac-glycerol for topical cosmetic compositions and applications.

### Example 2

This example demonstrates the anti-ageing benefits of the active ingredients of the present invention.

### Hydrogel Model for evaluation of rejuvenation activity.

Since YAP activity is highly dependent on substrate rigidity it was necessary to develop and perform assays on substrates with tunable mechanical properties. It has been reported in the literature that cells cultured on hydrogel substrates at ~ 2kPa or lower demonstrate negligible levels of nuclear YAP; therefore these materials are ideal for evaluating effect of YAP activators on rejuvenation phenotypes (such as promoting procollagen production and activation of genes important in building up the quantity and/or quality of the extracellular matrix of the dermal layer of skin).

Primary human dermal fibroblasts (P3) are plated at 31,579 cells per cm2 into 24-well 2kPa collagen coated hydrogel plates in 1ml 2% fetal bovine serum in Dulbecco's Modified Eagles Media (DMEM) and maintained 24 hours in a cell culture incubator at 5% carbon dioxide. The cells were washed twice with Dulbecco's Phosphate Buffered Saline then 1ml of DMEM supplemented with 0.6% fatty acid free bovine serum albumin. The cells were maintained for 24 hours in a cell culture incubator at 5% carbon dioxide.

Two hundred microliter of media was removed from each well. To each well were added 100ul of 500ug per ml ascorbic acid in DMEM supplemented with 0.6% fatty acid free bovine serum albumin. Test materials were diluted to 10X final concentration in DMEM supplemented with 0.6% fatty acid free bovine serum albumin or PBS supplemented with 0.1% fatty acid free bovine serum albumin and 100ul dosed to duplicate wells. The cells/plates were incubated for 3 days at 37°C, 5% CO₂ in a cell culture incubator. On day 3 the media was removed from the wells and 900ul DMEM supplemented with 0.6% fatty acid free bovine serum albumin plus 50ug/ml ascorbic acid. The treatments were added as described above. On day 6 after the start of treatment remove 100ul of the media from each well for the determination of the level of procollagen.

### Procollagen assay

The amount of procollagen in the media samples was determined using the Takara Bio Procollagen type 1 C-peptide (PIP) detection EIA assay. The assay was run following the manufacturer's instructions as following - 100ul of the reconstituted antibody-POD conjugate to the wells of the assay for the number required for the media samples and the standard curve. The procollagen standard solution was 2-fold serial diluted using sample diluent to generate a standard curve of 640, 320, 160, 80, 40, 20, 10 and 0 ng/ml procollagen. The media samples were diluted 1/20 with sample diluent. Twenty ul of the standard curve dilutions were added to the wells of the assay plate in duplicate. The 20ul media dilutions in sample diluent were added to the wells of the assay plate in singlet. The plate was incubated for 3 hours at 37°C, washed 4 times with phosphate buffered saline wash buffer and tapped to remove all residual wash buffer. One hundred ul of substrate solution (TMBZ) was added to each well and incubate 15 minutes. One hundred ul of stop solution was added to each well, the plate shaken to mix and the optical density at 450nm read in a plate reader. The amount of procollagen in the test samples was calculated from the standard curve values and corrected for the dilution factor and the volume in the well to generate the ng of procollagen in the samples. The amount of procollagen induced by the test materials was compared to the basal media.

The results in the table below demonstrate that ORG (within the scope of the present invention) surprisingly promotes the synthesis of procollagen-I in human dermal fibroblasts which is a known anti-ageing marker. In contrast, other molecules alone included in this assay, such as magnolol, hinokiol, oleoyl glycinate, and retinyl propionate, (which alone fall outside the scope of the present invention) do not exhibit such a pronounced effect in eliciting procollagen activity on substrates mimicking the biomechanical properties of skin.

**Table 4. Effect of Various Compound Treatments on Procollagen Synthesis in Human Dermal Fibroblasts**

| **Treatment** | **Procollagen ng/ml** | **Procollagen Std Dev** | **P-Value** |
|---|---|---|---|
| **Experiment 1** | | | |
| Control (Vehicle) | 1933.3 | 81.5 | |
| 30µM LPA | 3532.6 | 164.7 | 0.01 |
| 50µM ORG (Monomuls^{®}) | 3084.2 | 183.1 | 0.01 |
| 50 µM Oleoyl Glycinate | 2474.9 | 323.0 | 0.15 |
| 50 µM Hinokiol | 1946.1 | 25.16 | 0.85 |
| | | | |

| **Experiment 2** | | | |
|---|---|---|---|
| Control (Vehicle) | 2240.5 | 147.7 | |
| 30µM LPA | 3381.3 | 224.9 | 0.01 |
| 50µM ORG (Monomuls^{®}) | 3256.8 | 161.3 | 0.03 |
| 50 µM Magnolol | 2512.8 | 290.8 | 0.36 |
| 50 µM Hinokiol | 1843.5 | 161.3 | 0.12 |
| | | | |

| **Experiment 3** | | | |
|---|---|---|---|
| Control (Vehicle) | 1301.9 | 65.3 | |
| 50µM ORG (Monomuls^{®}) | 2275.4 | 26.8 | 0.00 |
| 50µM ORG (Monomuls^{®}) + 10 µM retinyl propionate | 2754.2 | 85.0 | 0.00 |
| 10 µM retinyl propionate | 1452.0 | 36.2 | 0.10 |

The boosting or maintenance of the level of procollagen 1 in skin is associated with many skin anti-ageing benefits such as wrinkle effacement and dermal repair of photodamaged skin.

Surprisingly, the combination of ORG and retinyl propionate resulted in a synergistic increase in procollagen production. The effect of the combination of ORG and retinyl propionate resulted in a statistically significant greater increase than either material alone, and the difference between ORG induction of procollagen was statically different than the ORG in combination with retinyl propionate.

### Activation of YAP downstream genes related to rejuvenation

Activation of YAP has been associated with regulation of downstream genes important for tissue regeneration in several organ systems, such as heart and intestine. Therefore, we sought to examine whether activation of YAP in our hydrogel model system also resulted in regulation of genes important for extracellular matrix buildup.

Primary human dermal fibroblasts (P3) are plated at 31,579 cells per cm² into 6-well 2kPa hydrogel collagen coated plates in 2ml 2% fetal bovine serum in Dulbecco's Modified Eagles Media (DMEM) and maintained 24 hours in a cell culture incubator at 5% carbon dioxide. The cells were washed twice with Dulbecco's Phosphate Buffered Saline DPBS) then 1ml of DMEM supplemented with 0.6% fatty acid free bovine serum albumin (FAF-BSA). The cells were maintained for 24 hours in a cell culture incubator at 5% carbon dioxide. Four hundred ul of media was removed from each well of the plates. Two hundred ul of the YAP active treatments diluted at 10X the desired final concentration plus 1% DMSO were diluted in DMEM supplemented with 0.6% fatty acid free bovine serum albumin. The control wells were treated with two hundred ul of 1% DMSO were diluted in DMEM supplemented with 0.6% fatty acid free bovine serum albumin. Two hundred ul of water-soluble YAP actives diluted at 10X the desired final concentration diluted in DPBS + 0.1% FAF-BSA. The controls wells were treated with 200ul DPBS + 0.1% FAF-BSA. The plates were incubated overnight at 37°C at 5% CO₂. In the AM the plates were dosed with the 220ul of the water-soluble YAP actives in DPBS + 0.1% FAF-BSA or the control well with 220ul DPBS + 0.1% FAF-BSA. After 6 hours the media was aspirate from the wells, the cells washed 2X with DPBS and the plate frozen at -80oC until the RNA was isolated.

### RNA Isolation -

The RNA was isolated using the Qiagen RNeasy mini kit following the manufacturer's instruction with the following modifications -

700 ul of RLT buffer plus beta-mercaptoethanol was added to each well immediately after removing the plate from the freezer. The plate was placed on an orbital shaker at 500 revolution per minute up for 15 minutes while the buffer thawed and extracted the cells. The cell lysate was transfer to Qiashedders and pass the Qiashedder to disrupt the cells. The recovery lysate was mixed with an equal volume of 70% Ethanol, 30% water. The RNA was purified on the minicolumns including the DNase step to remove DNA contamination. The RNA samples were quantitated on a NanoDrop 800 and quantity access on an Agilent Bio-analyzer. An aliquot of the RNA samples was reverse transcribed in cDNA then diluted to 5ng/ul of input RNA. The cDNA samples were evaluated for the expression level of select markers using comparative Taqman analysis using 2ul of the cDNA per reaction. The level of RNA expression was compared using the delta delta Cycle threshold (ddCt) method. Aliquot of the RNAs were also submitted to Lexogen (Vienna, Austria) for RNA-seq analysis.

**Table 5. Effect of ORG (Monomuls^{®}) on mRNA Gene Expression**

| | ORG (Monomuls^{®}) vs. Vehicle | |
|---|---|---|
| Gene: | Percent Increase | adj.P.Value |
| COL1A1 | 21.16 | 0.010 |
| COL3A1 | 15.35 | 0.014 |
| COL4A1 | 20.36 | 0.010 |
| COL5A1 | 10.73 | 0.086 |
| ELN | 18.49 | 0.004 |
| FN1 | 15.49 | 0.063 |
| FBN1 | 12.36 | 0.104 |
| HAS2 | 164.75 | 0.000 |

Shown in Table 5 is the average percent increase in mRNA expression for genes involved in extracellular matrix production following ORG treatment relative to the vehicle control, along with the Benjamini-Hochberg adjusted p-values (adj.p.value) calculated by converting the average log2fold-change (calculated using DESeq2) to a percentage increase using the following formula: Percent increase = 100*(2^log2FC)-100.

### Example 2A

An independent experiment was conducted using cell culture and RNA isolation methods described in Example 2 to further elucidate the retinyl propionate + ORG synergy (as observed in Table 4) by analyzing a well-described marker of a retinoid activity CRABPII (cellular retinoic acid binding protein II). After RNA was harvested from cells (as described in Example 2), an aliquot of the RNA sample was reverse transcribed to cDNA using High-Capacity RNA-to-cDNA Kit (Applied Biosystems^{™}). Then, 10ng cDNA samples per reaction were evaluated for the expression level of CRABPII using primers and comparative TaqMan^{™} Gene Expression Assay and TaqMan^{™} Fast Advanced Master (FAM) Mix (purchased from Applied Biosystems^{™}) and run in a ViiA^{™} 7 Real Time PCR System (from Applied Biosystems^{™}). Percent increases in CRABPII expression was calculated by converting the log2fold-change to a percentage increase using the following formula: Percent increase = 100*(2^log2FC)-100.

**Table 6**

| | **Percent Increase in CRABPII Gene Expression vs Vehicle** |
|---|---|
| 10 µM Retinyl Propionate | 138 |
| 50µM ORG (Monomuls ^{®}) | 54 |
| 50µM ORG (Monomuls ^{®}) + 10 µM Retinyl Propionate | 496 |

Retinoids play an important role in cell growth and differentiation and have been shown to be beneficial in counteracting skin aging. At the ultrastructural level, retinoids increase the thickness of the epidermis and have also been shown to boost the production of extracellular matrix molecules of the dermal layer such as collagen I, II, and VII as well as elastin and mucins. Retinoids are transferred to the cell nucleus by cellular retinoic acid binding proteins (CRABPs); CRABPII is the predominant isoform in skin. Given CRABPII's critical role in initiating retinoid cellular signaling, CRABPII expression is regarded as an early marker for retinoid activity and ensuing downstream skin rejuvenation benefits described above, such as, for example, increased collagen production.

As shown in Table 6, a retinoid, e.g., retinyl propionate, induces a robust increase in CRABPII gene expression, which was expected, while ORG produced a more modest increase in the retinoid biomarker. Surprisingly, the combination of ORG and retinyl propionate yielded a synergistic increase in CRABPII gene expression, further providing evidence of unexpected bioactivity offered by the combination of retinyl propionate and ORG. This unexpected and synergistic increase in CRABPII gene expression with the combination of retinyl propionate and ORG supports boosting of retinoid activity which is known to be important for skin anti-aging benefits, including but not limited to increased collagen production.

### Example 3

The formulation below describes an oil in water cream suitable for the methods and uses according to the present invention. The percentages indicated are by weight of the composition.

**Table 7**

| | **wt%** | **Wt%** | **Wt%** |
|---|---|---|---|
| Mineral Oil | 4 | 4 | 4 |
| ORG (MONOMULS^{®}) | 1.15 | 2 | 3 |
| cLPA | 2 | 1 | 0 |
| Retinyl Propionate | 0 | 1 | 2 |
| | | | |
| Brij 56* | 4 | 4 | 4 |
| Alfol 16RD* | 4 | 4 | 4 |
| Triethanolamine | 0.75 | 0.75 | 0.75 |
| Butane-1,3-diol | 3 | 3 | 3 |
| Xanthan gum | 0.3 | 0.3 | 0.3 |
| Perfume | Qs | qs | qs |
| Butylated hydroxy toluene | 0.01 | 0.01 | 0.01 |
| Water | to 100 | to 100 | To 100 |

| | | | |
|---|---|---|---|
| *Brij 56 is cetyl alcohol POE (10) Alfol 16RD is cetyl alcohol | | | |

### Example 4

The formulation below describes an emulsion cream according to the present invention.

**Table 8**

| **FULL CHEMICAL NAME OR CTFA NAME** | **TRADE NAME** | **WT.%** | **WT.%** | **WT %** |
|---|---|---|---|---|
| Disodium EDTA | Sequesterene Na2 | 0.05 | 0.05 | 0.05 |
| Magnesium aluminium silicate | Veegum Ultra | 0.6 | 0.6 | 0.6 |
| Methyl paraben | Methyl Paraben | 0.15 | 0.15 | 0.15 |
| Simethicone | DC Antifoam Emulsion | 0.01 | 0.01 | 0.01 |
| Butylene glycol 1,3 | Butylene Glycol 1,3 | 3.0 | 3.0 | 3.0 |
| Hydroxyethylcellulose | Natrosol 250HHR | 0.5 | 0.5 | 0.5 |
| Glycerine, USP | Glycerine USP | 2.0 | 2.0 | 2.0 |
| Xanthan gum | Keltrol 1000 | 0.2 | 0.2 | 0.2 |
| Triethanolamine | Triethanolamine (99%) | 1.2 | 1.2 | 1.2 |
| Stearic acid | Pristerene 4911 | 3.0 | 3.0 | 3.0 |
| Propyl paraben NF | Propylparaben NF | 0.1 | 0.1 | 0.1 |
| ORG | Monomuls^{®} | 1.5 | 2.0 | 3.0 |
| Stearyl alcohol | Lanette 18 DEO | 1.5 | 1.5 | 1.5 |
| Isostearyl palmitate | Protachem ISP | 6.0 | 6.0 | 6.0 |
| C12-15 alcohols octanoate | Hetester FAO | 3.0 | 3.0 | 3.0 |
| Dimethicone | Silicone Fluid 200 (50cts) | 1.0 | 1.0 | 1.0 |
| Cholesterol NF | Cholesterol NF | 0.5 | 0.5 | 0.5 |
| Sorbitan stearate | Sorbitan Stearate | 1.0 | 1.0 | 1.0 |
| Butylated hydroxytoluene | Embanox BHT | 0.05 | 0.05 | 0.05 |
| Tocopheryl acetate | Vitamin E Acetate | 0.1 | 0.1 | 0.1 |
| PEG-100 stearate | Myrj 59 | 2.0 | 2.0 | 2.0 |
| Sodium stearoyl lactylate | Pationic SSL | 0.5 | 0.5 | 0.5 |
| Hydroxycaprylic acid | Hydroxycaprylic Acid | 0.1 | 0.1 | 0.1 |
| Alpha-bisabolol | Alpha-bisabolol | 0.2 | 0.2 | 0.2 |
| Water, DI | | q.s. to 100 | q.s. to 100 | q.s. to 100 |

The above topical compositions provide an effective cosmetic treatment to improve the appearance of wrinkled, aged, photodamaged skin when applied to normal skin that has lost its smoothness and firmness through the normal aging process or when applied to youthful skin to help prevent or delay such undesirable changes. The compositions can be processed in conventional manner.

## Claims

1. A topical composition comprising:
(a) a cosmetic skin benefit agent selected from 1-oleoyl-rac-glycerol, (2S)-1-oleoylglycerol, oleoyl-rac-glycerol (ORG), (2R)-1-oleoylglycerol, 2-oleoylglycerol, and/or mixtures thereof;
(b) a retinoid, wherein the retinoid is retinyl propionate; and
(c) a dermatologically acceptable vehicle.

2. A topical composition according to claim 1, wherein the cosmetic skin benefit agent is 1-oleoyl-rac-glycerol.

3. A topical composition according to any of the preceding claims, wherein the cosmetic skin benefit agent is 2-oleoylglycerol.

4. A topical composition according to any of the preceding claims, wherein the composition further comprising cyclic phosphatidic acid (cLPA).

5. A cosmetic method of providing at least one cosmetic skin care benefit selected from:
treating/preventing wrinkling, sagging, dry, aged and/or photodamaged skin;
boosting/maintaining collagen levels in skin, boosting/maintaining decorin levels in skin, enhancing tissue repair; improving skin texture, smoothness and/or firmness; promoting skin barrier recovery;
the method comprising applying to the skin a topical composition as claimed in any preceding claim.

6. Use of a topical composition as claimed in any of claims 1 to 4 for providing at least one cosmetic skin care benefit selected from treating/preventing wrinkling, sagging, aged, dry, and/or photodamaged skin, boosting/maintaining collagen levels in skin, boosting/maintaining YAP levels in skin cell nucleus, enhancing tissue repair; improving skin texture, smoothness and/or firmness and/or promoting skin barrier recovery.

7. Use of oleoyl-rac-glycerol in combination with retinyl propionate as an activator of YAP activated receptors in a topical composition for providing at least one cosmetic skin care benefit selected from treating/preventing wrinkling, sagging, aged and/or photodamaged skin; boosting/maintaining collagen deposition in skin, boosting/maintaining decorin production in skin, enhancing tissue repair; improving skin texture, and smoothness and/or firmness and promoting skin barrier recovery.

8. A topical composition according to any of claims 1 to 4, wherein the cosmetic skin benefit agent is ORG or an oil with ORG.

## Patentansprüche

1. Topische Zusammensetzung, umfassend:
(a) einen kosmetischen Hautpflegewirkstoff, ausgewählt unter 1-Oleoyl-rac-Glycerin, (2S)-1-Oleoyl-Glycerin, Oleoyl-rac-Glycerin (ORG), (2R)-1-Oleoyl-Glycerin, 2-Oleoyl-Glycerin, und/oder Mischungen davon;
(b) ein Retinoid, wobei das Retinoid Retinylpropionat ist; und
(c) ein dermatologisch akzeptables Trägermittel.

2. Topische Zusammensetzung nach Anspruch 1, wobei der kosmetische Hautpflegewirkstoff 1-Oleoyl-rac-Glycerin ist.

3. Topische Zusammensetzung nach einem der vorhergehenden Ansprüche, wobei der kosmetische Hautpflegewirkstoff 2-Oleoyl-Glycerin ist.

4. Topische Zusammensetzung nach einem der vorhergehenden Ansprüche, wobei die Zusammensetzung zusätzlich cyclische Phosphatidsäure (cLPA) umfasst.

5. Kosmetisches Verfahren zur Bereitstellung mindestens eines kosmetischen Hautpflegevorteils, ausgewählt aus der Behandlung/Vorbeugung von Faltenbildung, Hauterschlaffung, trockener, gealterter und/oder lichtgeschädigter Haut;
Steigerung/Aufrechterhaltung des Kollagengehalts in der Haut, Steigerung/Aufrechterhaltung des Decoringehalts in der Haut, Verbesserung der Gewebereparatur; Verbesserung der Struktur, Glätte und/oder Festigkeit der Haut; Förderung der Wiederherstellung der Hautbarriere;
wobei das Verfahren das Auftragen einer topischen Zusammensetzung, wie in einem vorhergehenden Anspruch beansprucht, auf die Haut umfasst.

6. Verwendung einer topischen Zusammensetzung, wie in einem der Ansprüche 1 bis 4 beansprucht, zur Bereitstellung mindestens eines kosmetischen Hautpflegevorteils, ausgewählt aus der Behandlung/Vorbeugung von Faltenbildung, Hauterschlaffung, gealterter, trockener und/oder lichtgeschädigter Haut, Steigerung/Aufrechterhaltung des Kollagengehalts in der Haut, Steigerung/Aufrechterhaltung des YAP-Gehalts im Zellkern der Haut, Verbesserung der Gewebereparatur, Verbesserung der Struktur, Glätte und/oder Festigkeit der Haut und/oder Förderung der Wiederherstellung der Hautbarriere.

7. Verwendung von Oleoyl-rac-Glycerin in Kombination mit Retinylpropionat als Aktivator von YAP-aktivierten Rezeptoren in einer topischen Zusammensetzung zur Bereitstellung mindestens eines kosmetischen Hautpflegevorteils, ausgewählt aus der Behandlung/Vorbeugung von Faltenbildung, Hauterschlaffung, gealterter und/oder lichtgeschädigter Haut, Steigerung/Aufrechterhaltung der Kollagenablagerung in der Haut, Steigerung/Aufrechterhaltung der Decorinproduktion in der Haut, Verbesserung der Gewebereparatur; Verbesserung der Struktur und Glätte und/oder Festigkeit der Haut und Förderung der Wiederherstellung der Hautbarriere.

8. Topische Zusammensetzung nach einem der Ansprüche 1 bis 4, wobei der kosmetische Pflegewirkstoff ORG oder ein Öl mit ORG ist.

## Revendications

1. Composition à usage topique comprenant :
(a) un agent cosmétique bénéfique pour la peau choisi parmi le 1-oléoyl-rac-glycérol, le (2S)-1-oléoylglycérol, l'oléoyl-rac-glycérol (ORG), le (2R)-1-oléoylglycérol, le 2-oléoylglycérol, et/ou leurs mélanges ;
(b) un rétinoïde, lequel rétinoïde est le propionate de rétinyle ; et
(c) un véhicule acceptable en dermatologie.

2. Composition à usage topique selon la revendication 1, dans laquelle l'agent cosmétique bénéfique pour la peau est le 1-oléoyl-rac-glycérol.

3. Composition à usage topique selon l'une quelconque des revendications précédentes, dans laquelle l'agent cosmétique bénéfique pour la peau est le 2-oléoylglycérol.

4. Composition à usage topique selon l'une quelconque des revendications précédentes, laquelle composition comprend en outre de l'acide phosphatidique cyclique (cLPA).

5. Méthode cosmétique de fourniture d'au moins un bénéfice de soin cosmétique pour la peau choisi parmi :
le traitement/la prévention de la formation des rides, du relâchement, de la sécheresse, du vieillissement et/ou du photo-endommagement de la peau ;
le renforcement/le maintien des niveaux cutanés de collagène, le renforcement/le maintien des niveaux cutanés de décorine, l'amplification de la réparation tissulaire ; l'amélioration de la texture, du velouté et/ou de la fermeté de la peau ; l'amélioration de la restauration de la barrière cutanée ;
la méthode comprenant l'application sur la peau d'une composition à usage topique selon l'une quelconque des revendications précédentes.

6. Utilisation d'une composition à usage topique selon l'une quelconque des revendications 1 à 4 pour fournir au moins un bénéfice de soin cosmétique pour la peau choisi parmi le traitement/la prévention de la formation des rides, du relâchement, du vieillissement, de la sécheresse et/ou du photo-endommagement de la peau, le renforcement/le maintien des niveaux cutanés de collagène, le renforcement/le maintien des niveaux de YAP dans les noyaux des cellules cutanées, l'amplification de la réparation tissulaire ; l'amélioration de la texture, du velouté et/ou de la fermeté de la peau et/ou l'amélioration de la restauration de la barrière cutanée.

7. Utilisation d'oléoyl-rac-glycérol en combinaison avec du propionate de rétinyle en tant qu'activateur de récepteurs activés par YAP dans une composition à usage topique pour fournir au moins un bénéfice de soin cosmétique pour la peau choisi parmi le traitement/la prévention de la formation des rides, du relâchement, du vieillissement et/ou du photo-endommagement de la peau, le renforcement/le maintien du dépôt de collagène dans la peau, le renforcement/le maintien de la production de décorine dans la peau, l'amplification de la réparation tissulaire ; l'amélioration de la texture et du velouté et/ou de la fermeté de la peau et la promotion de la restauration de la barrière cutanée.

8. Composition à usage topique selon l'une quelconque des revendications 1 à 4, dans laquelle l'agent cosmétique bénéfique pour la peau est l'ORG ou une huile avec de l'ORG.
